# EUROPEAN PATENT SPECIFICATION

(11) **EP 1 263 348 B1**
(45) Date of publication and mention of the grant of the patent: **08.02.2006**
(21) Application number: 01912039.3
(22) Date of filing: 07.03.2001
(51) Int. Cl.: A61F 2/06

(54) **INTRALUMINAL PROSTHESIS**
INTRALUMINALE PROTHESE
PROTHESE INTRAVASCULAIRE

(30) Priority: 09.03.2000 US 522335
(43) Date of publication of application: 11.12.2002
(73) Proprietor: DESIGN & PERFORMANCE - CYPRUS LIMITED, Nicosia, 1066 (CY)
(72) Inventor: VONDERWALDE FREIDBERG, Carlos, Richmond, British Columbia V7C 4X5 (CA)
(74) Representative: Barz, Peter
(86) International application number: PCT/IB2001/000315
(87) International publication number: WO 2001/066037

(56) References cited:
- EP-A- 0 960 607
- WO-A-99/44536
- US-A- 5 935 161
- US-A- 6 004 347

## Description

### BACKGROUND OF THE INVENTION

This invention relates to the field of implantable medical devices, an more particularly to intraluminal grafts.

Stents are often used in conjunction with intravascular treatments such as ablation, atherectomy, balloon dilatation, and laser treatment for obstructive coronary and peripheral artery disease and other conditions. Stents are expandable, tubular devices which are positioned within a patient's vasculature or other body lumen, and which are typically expanded to implant the stent at a desired intraluminal position. Often, the stents are formed from a deformable metal and delivered to the desired intraluminal location by mounting the stent onto an expandable portion, e.g. a balloon, on the distal extremity of a catheter. By advancing the catheter through the body lumen, the stent may be delivered to a desired position and expanded therein by expanding the balloon to an expanded configuration, seating it within the artery or other body lumen. Other implementations make use of a self-expanding stent formed from a suitable material such as pseudoelastic material that is delivered in a constricted condition and when released spontaneously expands to an enlarged configuration. In other embodiments, a stent made of shape memory alloy (e.g. NiTi alloy) may be inserted into the body lumen in a martensitic phase and transformed to an austenite phase which has an expanded memory when raised to a temperature above the transformation temperature, usually less than 45°C. Further details of stents and stent delivery systems may be found in U.S. Patent Nos. 5,507,768 (Lau *et al.),* 5,458,615 (Klemm *et al*.), and 5, 514,154 (Lau *et al.).*

Covers, or grafts, have been provided on inner and outer surfaces of stents for a variety of purposes including inhibiting restenosis, treatment of aneurysms, saphenous vein grafts (SVG) and the remodeling or blocking of arteries. Restenosis, thought to be a natural healing reaction provoked by injury from the intravascular procedure, occurs in large percentage of patients who underwent percutaneous transluminal coronary angioplasty (PTCA) and other procedures. The healing process frequently causes thrombosis and may lead to intimal hyperplasia that occludes the vessel. Although helpful in reducing restenosis, stents do not represent a complete solution. The framework of the stent may still allow migration and proliferation of the smooth muscle cells, while the stent itself can be thrombogenic. To address these problems, stents have been provided with covers made from various materials such as DACRON®, polytetrafluoroethylene (PTFE), heterologous tissue and autologous veins, arteries and tissue.

In U.S. Patent 6,004,347 is taught a vascular prosthesis including a synthetic graft having porous walls so as to promote tissue ingrowth and one or two generally tubular anchors that are placed inside the lumen of the synthetic graft. Each tubular anchor is provided with spikes that hold the prosthesis in place by engaging the lumen of the organ in which the prosthesis is implanted. In some cases the anchors engage the synthetic graft by, when implanted, pushing the graft against the lumen of the organ in which the prosthesis is implanted. In some embodiments the anchors are attached to the synthetic graft.

It would be a significant advance to provide an intraluminal graft with an improved connection to a stent, and which facilitates implantation of the graft within a patient's body lumen. This invention satisfies these and other needs.

### SUMMARY OF THE INVENTION

The invention is directed to an intraluminal prosthesis, as claimed in claim 1, comprising a tubular graft having two support members secured thereto, each of the two support members configured to bend around an end of the tubular graft.

The intraluminal prosthesis generally comprises a tubular graft member having a first support member secured to the first end of the graft member and a second support member secured to the second end of the graft member. The support members allow the graft member secured thereto to be implanted in a body lumen with or without being attached to a stent.

The first and second support members each generally comprise a tubular body having a radially expandable configuration and a radially contractable configuration. A variety of different designs may be used to form the body of the support member, such as a sinusoidally wound wire or line, and other designs commonly used to form expandable intraluminal prostheses, such as triangular, zigzag, and other shapes. The support member is typically formed by shaping a wire or cutting a tube into an expandable and contractable member. The support member is preferably formed of metal, although other materials having sufficient strength and flexibility may also be used. Optionally, additional support members may be secured to the graft member. At least one intermediate member may optionally be provided extending between the support members and connecting the support members together. In one embodiment, eyelet members are provided on.the support members having an opening configured to receive a securing member such as a suture to secure the graft member to the support members.

The graft member generally comprises a tubular body. It may be a formed of a variety of suitable materials which are preferably expandable, biocompatible, and non-thrombogenic, including autologous tissue, heterologous tissue such as bovine pericardium, porcine pericardium, aortic leaflet, and polymeric materials such as polytetrafluoroethylene (PTFE), expanded PTFE, and polyesters such as DACRON®. In a preferred embodiment, heterologous pericardium forms the graft member. It is typically a solid walled material which does not allow proliferation of cells through the graft walls and into the lumen of the graft. In a preferred embodiment, the graft member is generally cylindrical for corresponding to the tubular framework of the stent.

The graft member may be secured to the support members using a variety of suitable securing members such as hooks, piercing members, clamps, sutures, adhesive, staples, bending members, and the like. The graft member and the support members are in surrounding relation to one another, so that the support members are secured to an inner surface or to an outer surface of the graft member. In one embodiment, the support members are configured to bend around the end of the graft member, so that the support member is attached to either an inner or an outer surface of the graft member and is bent around the end of the graft member into contact with either the graft member outer or inner surface, respectively.

The support members can be radially contracted to crimp onto or otherwise attach to a stent, to form a stent assembly. Additionally, after crimping onto the stent, the support members can be further attached to the stent with sutures, welding, clamped of hinged members, and the like. The stent may be an expandable, tubular framework and may be a conventional self expanding or balloon expandable stent. A variety of stent designs may be used, including stents formed from braided tubes, slotted tubes, and coils or closed sinusoidal rings of wire or ribbon.

The intraluminal prosthesis of the invention is implanted in a patient's body lumen, such as a blood vessel, typically by mounting the prosthesis or stent assembly on the distal extremity of a delivery catheter. In the case of the stent assembly, the support members secured to the graft member are attached onto a stent, preferably including crimping by applying radially compressive pressure onto the support members, and with sutures, clamps, welds or other attachment members. The prosthesis or stent assembly is mounted on a balloon, for a balloon expandable support member or stent, or on a containing mechanism for a self expandable support member or stent. The catheter is advanced through the patient's body lumen until the distal extremity of the catheter having the graft or stent assembly is positioned at a desired location therein. The prosthesis or stent assembly is expanded by expanding the balloon or releasing the containing mechanism on which it is mounted to anchor the prosthesis or stent assembly within the body lumen. Once the prosthesis or stent assembly is effectively positioned within the body lumen, the expanded balloon may be contracted, e.g. by deflation, and then the delivery catheter may be withdrawn.

The support members of the invention provide for easy attachment of a graft onto a stent. This is particularly advantageous in the case of a graft member which is impregnated with a therapeutic or diagnostic agent, such as an angiogenesis or antithrombotic agent, just before use or stored in the agent to preimpregnate the graft member. The support members allow the impregnated graft member to be quickly and easily attached to the stent just before implantation of the prosthesis or stent assembly in the patient's body lumen. Thereafter, the prosthesis or stent assembly can be positioned at a desired site within the patient's body lumen, where the graft member will release the therapeutic agent.

The support members, secured to a graft member, of the invention allow the graft member to be implanted in a body lumen without the use of an underlying stent, or alternatively, provide an easily formed and low profile connection between the graft member and a stent. These and other advantages of the invention will become more apparent from the following detailed description and exemplary figures.

### BRIEF DESCRIPTION OF THE DRAWINGS

Figure 1 is a perspective view of an intraluminal prosthesis which embodies features of the invention, having support members, shown in phantom, on an inner surface of a graft member.
Figure 2 is a perspective view of a stent assembly, illustrating a stent disposed within a lumen of the prosthesis shown in Fig. 1.
Figure 3 is an elevational view illustrating the stent assembly shown in Fig. 2, partially in longitudinal cross section.
Figure 4 is a perspective view of an intraluminal prosthesis, having support members on an outer surface of a graft member, and having intermediate members connecting the support members.
Figure 5 is an elevational view illustrating the prosthesis shown in Fig. 4, partially in longitudinal cross section.
Figure 6 is an elevational view of a section of a support member having a closed ring eyelet for receiving a securing member to secure the graft member to the support member.
Figure 7 is an elevational view of a section of a support member having an open ring eyelet for receiving a securing member to secure the graft member to the support member.
Figure 8 is an elevational view of a section of a support member having a cover connector with two piercing ends.
Figure 9 is an elevational view of a section of a support member having a cover connector with a single piercing end.
Figure 10 is an elevational view, partially in longitudinal cross section, of a stent assembly having an intraluminal prosthesis which embodies the invention, having support members bent around the graft member ends.

### DETAILED DESCRIPTION OF THE INVENTION

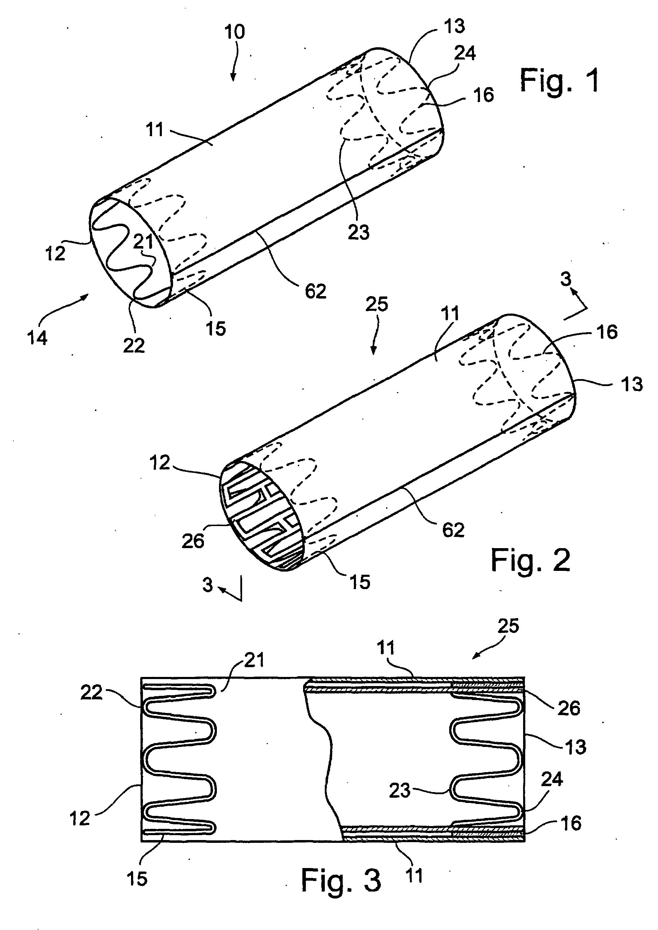

Fig. 1 illustrates a perspective view of an intraluminal prosthesis 10, generally comprising a tubular graft member 11 having a first end 12, a second end 13, and a lumen 14 extending from the first end to the second end, and a first support member 15 secured to the first end 12 of the graft member 11, and a second support member 16 secured to the second end 13 of the graft member 11. In the embodiment illustrated in Fig. 1, the support members 15/16 are inside the graft member lumen 14, so that the graft member 11 is in surrounding relation to the support members 15/16. At least a section of the support members 15/16 is secured to an inner surface of the graft member 11 which defines the graft member lumen 14. The first support member 15 has a first end 21 and a second end 22 which is opposite the first end and which is aligned with the first end 12 of the graft member 11. Similarly, the second support member 16 has a first end 23 and a second end 24 which is opposite the first end and which is aligned with the second end 13 of the graft member 11. In the embodiment illustrated in Fig. 1, each support member 15/16 comprises a tubular body configured to contract to a radially compressed configuration or expand to a radially enlarged configuration.

Fig. 2 illustrates a perspective view of a stent assembly 25, generally comprising the prosthesis 10 shown in Fig. 1 and a stent 26 therein. The stent 26 typically comprises an expandable tubular body having first and second ends and a lumen therein. As best illustrated in Fig. 3, illustrating a partial longitudinal cross section of the stent assembly shown in Fig. 2, the first support member 15 is radially compressed onto an outer surface of the first end of the stent expandable tubular body, and the second support member 16 is radially compressed onto an outer surface of the second end of the stent expandable tubular body, to thereby secure the first and second support members 15/16 and graft member 11 to the stent 26. The stent could alternatively be radially compressed onto an outer surface of the support members 15/16.

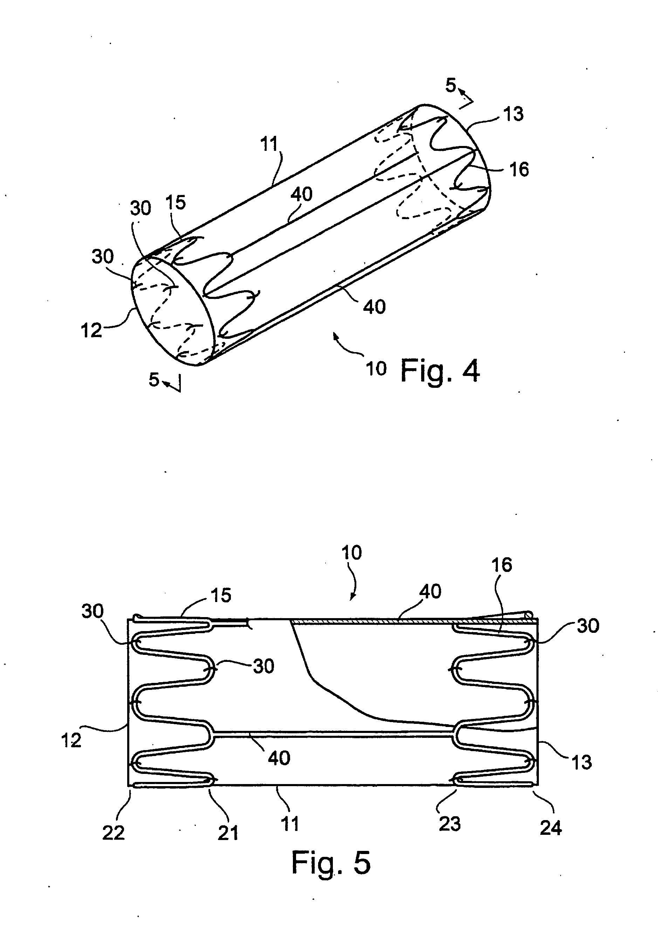

Figs. 4 and 5 illustrate an alternative embodiment, in which the first and second support members 15/16 are secured to an outer surface of the graft member 11. Fig. 4 illustrates a perspective view of prosthesis 10 having support members 15/16 on an outer surface, and Fig. 5 illustrates an elevational view, partially in longitudinal cross section, of the prosthesis 10 shown in Fig. 4. Although not illustrated, it should be understood that the embodiment of the prosthesis 10 illustrated in Figs. 4 and 5 may be secured to a stent to form a stent assembly as discussed above in relation to the embodiment of Fig. 3.

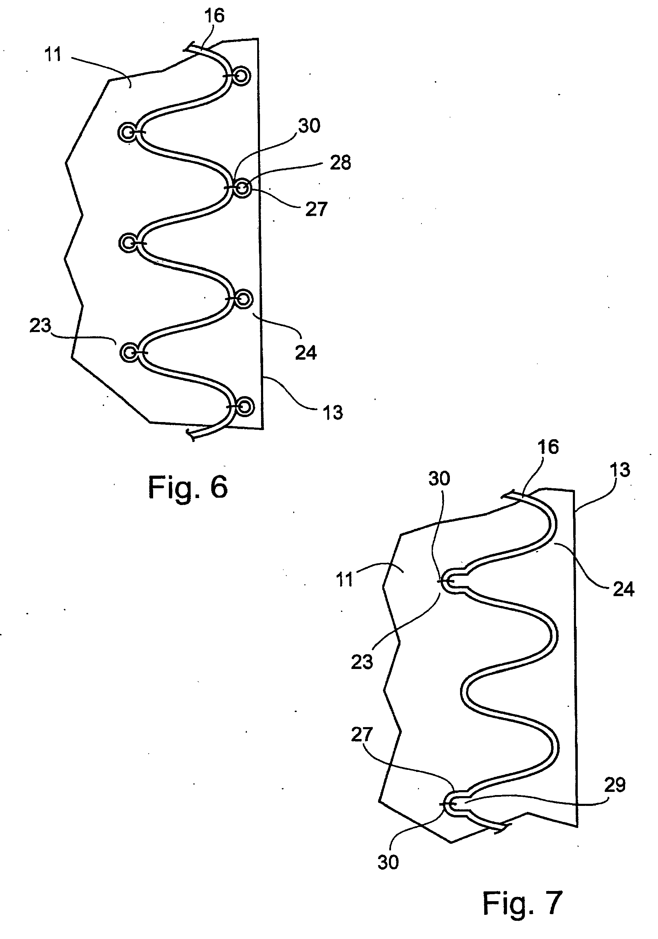

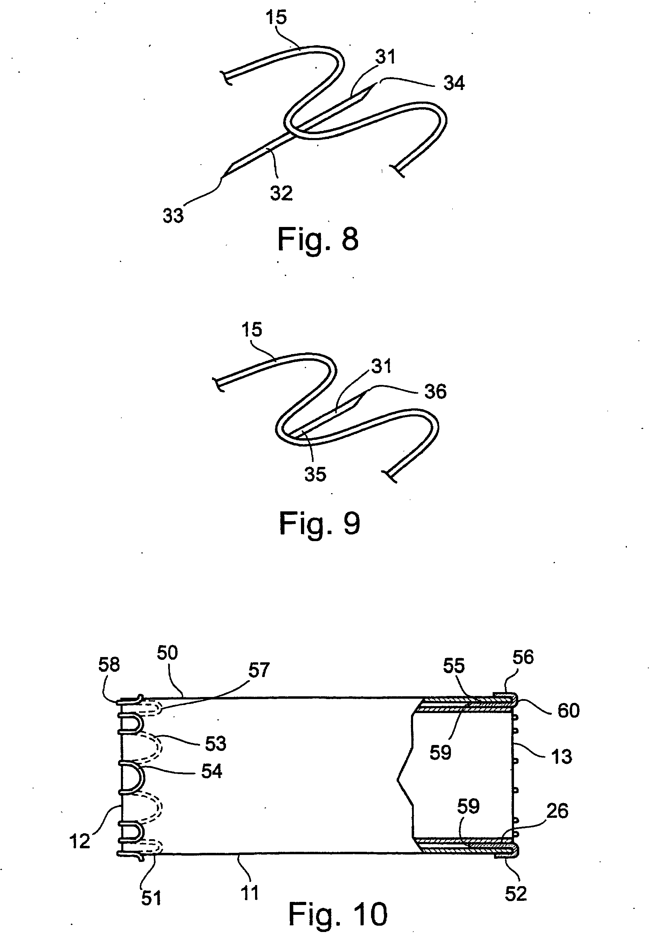

A variety of suitable securing members may be used to secure the support members to the graft member. In the embodiment illustrated in Fig. 4, sutures 30 secure the support members to the graft member. Sutures 30 are provided at one or more locations on the support members. Typically, sutures 30 are provided at multiple locations around the circumference of each support member. In one embodiment, eyelets 27 are provided on the support members for receiving sutures 30, or other securing members such as staples, hooks, and wires. Fig. 6 illustrates one embodiment in which eyelets 27 are closed rings or loops extending from the side of the body of the support member, on the ends of the sinusoidal turns of the support member 15, with a central opening 28 configured to receive suture 30, to secure the support member 15 to the graft member 11. In an alternate embodiment, eyelets 27 are open rings or loops, as for example with an aperture 29 adjacent to the edge of the turns of the support member 15 and with an opening 28 configured to receive suture 30 illustrated in Fig. 7. In the embodiment of Fig. 6, eyelets 27 are on the outer edge of each sinusoidal turn of the support member on both sides of the support member. However, in alternative embodiments, the eyelets 27 may be provided on less than all of the turns of support member, as for example, on alternate turns and on only the turns on one side of the support member, as illustrated in Fig. 7. In a presently preferred embodiment, eyelets 27 are provided around the circumference of the support members 15/16. However, in an alternative embodiment, the eyelets 27 may be provided only along a narrow section of the support members along the length of the support members, as for example where a seam of the tubular graft is located. A variety of suitable materials may be used to form the eyelets 27, including stainless steel, nitinol, and tantalum. In a presently preferred embodiment, eyelets 27 are formed of the same material as support members. Eyelets 27 may be formed integrally with the support members, or alternatively, as a separate member secured to the support member, as for example by welding. Eyelets preferably have the same thickness as the support members. In alternative embodiments, the securing members comprise cover connectors 31, as illustrated in Figs. 8 and 9. The cover connectors 31 generally comprise a pointed projection attached to the support member, configured to pierce through the graft member 11 to thereby secure the graft member 11 to the support member. In the embodiment illustrated in Fig. 8, cover connector 31 is a body 32 in an open configuration with a first end section having a first pointed end 33 and a second end section having a second pointed end 34, which are configured to pierce the graft member 11 and bend together into a closed configuration which retains the graft member on the cover connector body 32. In the embodiment illustrated in Fig. 9, cover connector 31 is a body 35 in an open configuration with an end section with a single pointed end 36 configured to pierce graft member and bend back into a closed configuration which retains the graft member on the cover connector body 35. For ease of illustration, securing members which secure the graft member 1-1 to the support members 15/16 are not illustrated in the embodiment shown in Fig. 1. However, the graft member can be secured to the support members using a variety of suitable securing members, as illustrated in Figs. 4, 8 and 9, and as discussed above.

In the embodiment illustrated in Fig. 4, a plurality of intermediate members 40 are secured to and extend between the first and second support members 15/16 along an outer surface of the graft member 11. Intermediate members 40 connect the first and second support members 15/16 together and maintain a fixed length between the support members, and are configured to prevent or inhibit the collapsing or turning up of the graft member 11 particularly during advancement of the prosthesis within the patient's vasculature. In the illustrated embodiment, the intermediate members 40 comprise a line or rod, longitudinally aligned with the graft member 11 longitudinal axis. In alternative embodiments (not shown), the intermediate member may have an alternative shape such as sinusoidal, curved, zigzag, and the like. Preferably, intermediate members are made of stainless steel, however a variety of suitable materials may be used. In a presently preferred embodiment, the number of intermediate members is two, although one or more intermediate members may be used.

Fig. 10 illustrates an intraluminal prosthesis of the invention, generally comprising a graft 50 having a first support member 51 and a second support member 52, each configured to bend around an end of the graft member 11. Features otherwise the same as in the previous embodiments have the same reference numerals. In the embodiment illustrated in Fig. 10, showing an elevational view, partially in longitudinal cross section, graft 50 is on a stent 26, to form a stent assembly. First support member 51 has a first section 53 secured to an inner surface of the graft member 11, and a second section 54 bent around the graft member end to contact at least a portion of an outer surface of the graft member 11 opposite to the inner surface of the graft member 11. Similariy, the second support member 52 has a first section 55 secured to the graft member inner surface, and a second section 56 bent around the graft member end into contact with the graft member outer surface. In an alternative embodiment, the second section 54/56 may be secured to the graft member outer surface and the first section 53/55 bent around into contact with the graft member inner surface. In another alternative embodiment, the ends of the support members 51/52 are bent around the end of the graft without separately securing a section of the support member 51/52 to one side of the graft. The first support member 51 has a first edge 57 and a second edge 58 opposite to the first edge 57 and adjacent to the first end 12 of the graft member 11. The second support member 52 has a first edge 59 and a second edge 60 opposite to the first edge 59 and adjacent to the second end 13 of the graft member 11. The support members 51/52 bend around the ends of the graft member 11, so that the first end 12 of the graft member does not extend beyond the second edge 58 of first support member 51, and second end 13 of graft member 11 does not extend beyond the second edge 60 of the second support member 52. Although illustrated as a stent assembly, it should be understood that graft 50 may be implanted in a patient without stent 26.

The support members 15/16 each preferably has a length less than about 1 to about 50%, most preferably less than about 10 to about 30% for coronary applications, of the length of the graft member 11. The length of support members 15/16 are about 0.5 to about 300 mm, and the length is most preferably about 2 to about 4 mm for coronary applications. The expanded outer diameter of support members 15/16 is about 1 to about 50 mm, and the length is most preferably about 2 to about 5 mm for coronary applications. The support members 15/16 are preferably formed of a metallic material such as stainless steel. However, other resilient materials can be used including platinum or nickel-titanium alloy such as nitinol, and tantalum, or other suitable material such as plastic.

The graft member 11 is preferably a biocompatible, non-thrombogenic material, such as autologous and/or heterologous tissue, PTFE, or DACRON®. As illustrated in Fig. 1, graft member 11 has a seam 62 formed from the wrapping of a sheet of material into a cylinder to form tubular graft member 11. The thickness of the graft member 11 is typically from about 0.07 mm to about 1 mm, and preferably is about 0.1 mm to about 0.4 mm. The graft member 11 preferably has a length configured to cover the length of the expanded stent 26, as illustrated in Fig. 3, showing a stent 26 with a graft member 11 extending the length of the stent, with a length equal to the stent length. However, the graft member 11 may have a length that is less than or greater than the length of the stent. The graft member 11 preferably has a circumference about equal to the circumference of the expanded stent 26 and support members 15/16, to preferably fit on the expanded stent 26 and support members 15/16 so that the graft member 11 conforms to the expanded stent 26 and support members 15/16 without flaps of excess material.

Stent 26 is typically a metallic material and may comprise a variety of suitable stent designs. Thus, in the embodiment where the graft is secured to a stent 26 to form a stent assembly, a variety of commercially available stents may be used such as GFX, GFX2 stents available from Arterial Vascular Engineering (AVE), S670 available from Medtronic/AVE, and Multi-Link® duet available from Guidant, and Nir® stents available from Boston Scientific. Other stents that may be used in the practice of this invention include the Palmaz-Shatz® stent from Johnson and Johnson, the Gianturco® stent from Cook Incorporated and other commercially available stents. Conventional balloon expandable stents are preferred, but self-expanding stents, such as those formed from shape memory materials, are also suitable. The length of the stent, for coronary applications, is generally about 4 to greater than about 80 mm, typically about 5 to about 80 mm, preferably about 10 to about 50 mm. The stent generally has a diameter of about 1.5 to about 35 mm, typically about 2 to about 6 mm, preferably about 2.5 to about 5 mm. The actual length and diameter of the stent and graft member may vary, and will depend on the nature of the vessel in which the stent assembly is implanted. For example, for peripheral vessel applications, such as an aortic abdominal aneurysm, a larger stent having a length of about 5 mm to about 200 mm and a diameter of about 2 mm to about 60 mm would be used.

A method of securing an intraluminal prosthesis to a stent generally comprises providing a tubular graft member having a first end and second end and a lumen therein, securing a first support member to the first end, and securing a second support member to the second end, and providing a stent comprising an expandable tubular body having first and second ends and a lumen therein, and securing the tubular graft member secured to the first and second support members to an outer surface of the stent by radially compressing the first support member and the second support member onto the stent. In one embodiment, the support members are secured to the graft member by bending a second section of the support members around the graft member end into contact with at least a portion of the inner or outer surface of the graft member.

Graft 10/50 of the invention may be implanted in the patient without stent 26, as for example in procedures such as remodeling or blocking off of arteries. A method of performing a medical procedure generally comprises introducing into a patient's body lumen an intraluminal graft on a catheter, the intraluminal graft comprising a tubular graft member having a first end, a second end, and a lumen extending from the first end to the second end, and a first support member secured to the first end of the tubular graft member, and a second support member secured to the second end of the tubular graft member, and expanding the first and second support members to secure the intraluminal graft within the patient's body lumen. In one embodiment, the catheter is a balloon catheter. When a stent 26 is not provided, the intraluminal graft is directly mounted on the balloon, and the balloon is inflated to expand the support members and the graft member secured thereto.

Although primarily described with respect to preventing restenosis in angioplasty patients, the graft of this invention may be used in a number of coronary artery, peripheral artery and non-vascular applications, including artery remodeling and blocking arteries, and treatment of aneurysms. For example, coronary artery applications include use in ectatic arteries and ectatic arteries containing an obstructive lesion, aneurismatic arteries, saphenous vein grafts and native arteries, coronary perforation, coronary fistula, and ostial coronary lesions. Peripheral artery applications include aortic abdominal aneurysm and other aneurismatic peripheral arteries, transjugular intrahepatic portal shunt, percutaneous transluminal angioplasty, fistula closing and neuro interventions (such as aneurysms and arterial-venous malformations), small vessel intraluminal grafting, and ostial renal artery lesions. Additionally, the intraluminal prosthesis of this invention may be used in urological, gastroenterological, respiratory, neurological, and other non-vascular applications. For example, urological field applications include urethral stenting for stenosis due to tumors, fibrous tissue and perforation. Gastroenterological field applications include fistula closing, reconstruction such as esophagus reconstruction, and esophageal bleeding. Respiratory field applications include tracheal and bronchial obstructions, and neurological field applications include carotid angioplasty.

A general description of the device of the present invention as well as a preferred embodiment of the present invention has been set forth above. One skilled in the art will recognize that a variety of modifications may be made within the scope of the invention. For example, although the graft member is illustrated primarily in terms of a sheet of material forming a cylinder, a variety of the graft member may be used such as ribbons of material wrapped in whole or in part about itself. Additionally, although individual features may be shown and discussed in terms of one embodiment, they may be applied to the other embodiments of the invention. For example, although intermediate members 40 are illustrated in the embodiment shown in Fig. 4, they may also be provided in the embodiments of Figs. 1 and 10.

## Claims

1. An intraluminal prosthesis (50), comprising:
a) a tubular graft member (11) having a first graft member end (12), a second graft member end (13), and a lumen (14) extending from said first graft member end (12) to said second graft member end (13); and
b) a first support member (51) secured to said first graft member end (12), and a second support member (52) secured to said second graft member end (13)
wherein said first support member (51) and said second support member (52) each have a first section (53, 55) secured to one of an inner surface or an outer surface of said tubular graft member (11), and a second section (54, 56) configured to bend around a graft member end (12, 13) so as to contact at least a portion of a surface of said tubular graft member (11) opposite to said one surface of said tubular graft member (11) to which said first section (53, 55) is secured, where for each support member (51, 52) a said first section (53, 55) and a said second section (54, 56) each has a first end (57, 59) and a common second end (58, 60), where a graft member end (12, 13) does not extend beyond a said common second end (58, 60) of a support member (51, 52) around which a said support member (51, 52) is bent.

2. The intraluminal prosthesis of claim 1, wherein said tubular graft member (11) is of a solid walled material which does not allow proliferation of cells through walls of said graft tubular graft member.

3. The intraluminal prosthesis (50) of claim 1, wherein said first support member (51) and said tubular graft member (11) are in surrounding relation to one another, and said second support member (52) and said tubular graft member (11) are in surrounding relation to one another.

4. The intraluminal prosthesis (50) of claim 3, wherein said first support member (51) and said second support member (52) each have a first support member end (57, 59) and a second support member end (58, 60) opposite to a said first support member end (57, 59) of a respective support member (51, 52), and where said second support member end (57, 59) is aligned with a graft member end (12, 13).

5. The intraluminal prosthesis (50) of claim 1, wherein said graft member (11) is secured to said first support member (51) and said second support member (52) with securing members (30, 31) selected from the group consisting of hooks, piercing members, clamps, sutures, staples, adhesive, and bending members.

6. The intraluminal prosthesis (50) of claim 1, wherein at least a section of said first support member (51) and at least a section of said second support member (52) are secured to an inner surface of said graft member (11), said graft member (11) inner surface defining a graft member lumen (14), or at least a section of said first support member (51) and at least a section of said second support member (52) are secured to an outer surface of said tubular graft member (11).

7. The intraluminal prosthesis (50) of claim 1, devoid of an intermediate member connecting between said first support member (51) and said second support member (52).

8. The intraluminal prosthesis (50) of claim 1, including at least one intermediate member (40) secured to and extending between said first support member (15, 51) and said second support member (16, 52) along a surface of said graft member (11), and connecting said first support member (15, 51) to said second support member (16, 52).

9. The intraluminal prosthesis (50) of claim 1, wherein said graft member (11) comprises a material selected from the group consisting of tissue, autologous tissue, heterologous tissue, pericardium, bovine pericardium, porcine pericardium, aortic leaflet, veins, arteries, polymeric materials, polytetrafluoroethylene, expanded polytetrafluoroethylene and polyesters.

10. The intraluminal prosthesis (50) of claim 1, wherein said support members (51, 52) have a length not greater than 50% of the length of the graft member (11).

11. The intraluminal prosthesis (50) of claim 1, wherein said support members (51, 52) have a length of about 0.5 mm to about 300 mm.

12. The intraluminal prosthesis (50) of claim 1, wherein each of said support members (51, 52) comprises a substantially tubular body having a radially compressed configuration and a radially expanded configuration.

13. The intraluminal prosthesis (50) of claim 1, including at least one eyelet member (27) on a said support member (51, 52), said at least one eyelet member (27) having an aperture (29) configured to receive a securing member (30) therein to secure said graft member (11) to said support member (51, 52).

## Patentansprüche

1. Intraluminale Prothese (50), umfassend:
a) ein rohrförmiges Pfropfenelement (11) mit einem ersten Pfropfenelementende (12), einem zweiten Pfropfenelementende (13) und einem Lumen (14), das sich von dem ersten Pfropfenelementende (12) zu dem zweiten Pfropfenelementende (13) erstreckt; und
b) ein erstes Trägerelement (51), das an dem ersten Pfropfenelementende (12) befestigt ist, und ein zweites Trägerelement (52), das an dem zweiten Pfropfenelementende (13) befestigt ist, wobei das erste Trägerelement (51) und das zweite Trägerelement (52) jeweils aufweisen: einen ersten Abschnitt (53, 55), der an einer inneren Oberfläche oder einer äußeren Oberfläche des rohrförmigen Pfropfenelements (11) befestigt ist, und einen zweiten Abschnitt (54, 56), der konfiguriert ist, um sich um ein Pfropfenelementende (12, 13) zu biegen, um mindestens einen Abschnitt einer Oberfläche des rohrförmigen Pfropfenelements (11) zu kontaktieren, der der besagten einen Oberfläche des rohrförmigen Pfropfenelements (11) gegenüberliegt, an der der erste Abschnitt (53, 55) befestigt ist, wobei für jedes Trägerelement (51, 52) ein besagter erster Abschnitt (53, 55) und ein besagter zweiter Abschnitt (54, 56) jeweils ein erstes Ende (57, 59) und ein zweites gemeinsames Ende (58, 60) aufweisen, wobei sich ein Pfropfenelementende (12, 13) nicht über ein besagtes gemeinsames Ende (58, 60) eines Trägerelements (51, 52) erstreckt, um das ein besagtes Trägerelement (51, 52) gebogen ist.

2. Intraluminale Prothese gemäß Anspruch 1, bei der das rohrförmige Pfropfenelement (11) aus einem vollwandigen Material ist, das keine Proliferation von Zellen durch Wände des pfropfenrohrförmigen Pfropfenelements ermöglicht.

3. Intraluminale Prothese (50) gemäß Anspruch 1, bei der das erste Trägerelement (51) und das rohrförmige Pfropfenelement (11) in umgebenden Beziehung zueinander sind, und das zweite Trägerelement (52) und das rohrförmige Pfropfenelement (11) in umgebender Beziehung zueinander sind.

4. Intraluminale Prothese (50) gemäß Anspruch 3, bei der das erste Trägerelement (51) und das zweite Trägerelement (52) jeweils ein erstes Trägerelementende (57, 59) und ein zweites Trägerelementende (58, 60), das einem besagtem ersten Trägerelementende (57, 59) eines jeweiligen Trägerelements (51, 52) gegenüberliegt, aufweisen, und wobei das zweite Trägerelementende (57, 59) mit einem Pfropfenelementende (12, 13) ausgerichtet ist.

5. Intraluminale Prothese (50) gemäß Anspruch 1, bei der das Pfropfenelement (11) an dem ersten Trägerelement (51) und dem zweiten Trägerelement (52) mit Befestigungselementen (30, 31) befestigt ist, die aus der Gruppe ausgewählt sind, die aus Haken, durchstechenden Elementen, Klemmen, Nähten, Klammern, Kleber und Biegeelemente besteht.

6. Intraluminale Prothese (50) gemäß Anspruch 1, bei der mindestens ein Abschnitt des ersten Trägerelements (51) und mindestens ein Abschnitt des zweiten Trägerelements (52) an einer inneren Oberfläche des Pfropfenelements (11) befestigt sind, wobei die innere Oberfläche des Pfropfenelements (11) ein Pfropfenelement-Lumen (14) definiert, oder mindestens ein Abschnitt des ersten Trägerelements (51) und mindestens ein Abschnitt des zweiten Trägerelements (52) an einer äußeren Oberfläche des rohrförmigen Pfropfenelements (11) befestigt sind.

7. Intraluminale Prothese (50) gemäß Anspruch 1, ohne ein Zwischenelement, das zwischen dem ersten Trägerelement (51) und dem zweiten Trägerelement (52) verbindet.

8. Intraluminale Prothese (50) gemäß Anspruch 1, mit mindestens einem Zwischenelement (40), das an dem ersten Trägerelement (15, 51) und dem zweiten Trägerelement (16, 52) entlang einer Oberfläche des Pfropfenelements (11) befestigt ist und sich dazwischen erstreckt und das das erste Trägerelement (15, 51) mit dem zweiten Trägerelement (16, 52) verbindet.

9. Intraluminale Prothese (50) gemäß Anspruch 1, bei der das Pfropfenelement (11) ein Material umfasst, das aus der Gruppe ausgewählt wird, die aus Gewebe, autologem Gewebe, heterologem Gewebe, Pericardium, Rinder-Pericardium, Schweine-Pericardium, Aortensegel, Venen, Arterien, polymeren Materialien, Polytetrafluorethylen, geschäumtem Polytetrafluorethylen und Polyestern besteht.

10. Intraluminale Prothese (50) gemäß Anspruch 1, bei der die Trägerelemente (51, 52) eine Länge aufweisen, die nicht größer als 50% der Länge des Pfropfenelements (11) ist.

11. Intraluminale Prothese (50) gemäß Anspruch 1, bei der die Trägerelemente (51, 52) eine Länge von etwa 0,5 mm bis etwa 300 mm aufweisen.

12. Intraluminale Prothese (50) gemäß Anspruch 1, bei der jedes der Trägerelemente (51, 52) einen im Wesentlichen rohrförmigen Körper umfasst, der eine radial komprimierte Konfiguration und eine radial expandierte Konfiguration aufweist.

13. Intraluminale Prothese (50) gemäß Anspruch 1, mit mindestens einem Ösenelement (27) auf einem besagten Trägerelement (51, 52), wobei das mindestens eine Ösenelement (27) eine Öffnung (29) aufweist, die konfiguriert ist, um ein Befestigungselement (30) darin aufzunehmen, um das Pfropfenelement (11) an dem Trägerelement (51, 52) zu befestigen.

## Revendications

1. Prothèse endovasculaire (50), comprenant :
a) un élément formant greffon tubulaire (11) ayant une première extrémité d'élément greffon (12), une deuxième extrémité d'élément greffon (13) et une lumière (14) qui s'étend de ladite première extrémité d'élément greffon (12) à ladite deuxième extrémité d'élément greffon (13) ; et
b) un premier élément de support (51) fixé à ladite première extrémité d'élément greffon (12), et un deuxième élément de support (52) fixé à ladite deuxième extrémité d'élément greffon (13),
dans laquelle ledit premier élément de support (51) et ledit deuxième élément de support (52) ont chacun une première section (53, 55) fixée soit à une surface intérieure, soit à une surface extérieure dudit élément formant greffon tubulaire (11), et une deuxième section (54, 56) conçue pour se replier autour d'une extrémité d'élément greffon (12, 13) afin de toucher au moins une partie d'une surface dudit élément formant greffon tubulaire (11) opposée à ladite une surface dudit élément formant greffon tubulaire (11) à laquelle est fixée ladite première section (53, 55), où pour chaque élément de support (51, 52) une dite première section (53, 55) et une dite deuxième section (54, 56) a chacune une première extrémité (57, 59) et une deuxième extrémité commune (58, 60), où une extrémité d'élément greffon (12, 13) ne s'étend pas au-delà d'une dite deuxième extrémité commune (58, 60) d'un élément de support (51, 52) autour de laquelle un dit élément de support (51, 52) est recourbé.

2. Prothèse endovasculaire selon la revendication 1, dans laquelle ledit élément formant greffon tubulaire (11) est fait d'un matériau à paroi pleine qui ne permet pas la prolifération de cellules à travers les parois dudit élément formant greffon tubulaire.

3. Prothèse endovasculaire (50) selon la revendication 1, dans laquelle ledit premier élément de support (51) et ledit élément formant greffon tubulaire (11) sont en relation enveloppante l'un par rapport à l'autre, et ledit deuxième élément de support (52) et ledit élément formant greffon tubulaire (11) sont en relation enveloppante l'un par rapport à l'autre.

4. Prothèse endovasculaire (50) selon la revendication 3, dans laquelle ledit premier élément de support (51) et ledit deuxième élément de support (52) ont chacun une première extrémité d'élément de support (57, 59) et une deuxième extrémité d'élément de support (58, 60) opposée à une dite première extrémité d'élément de support (57, 59) d'un élément de support respectif (51, 52), et où ladite deuxième extrémité d'élément de support (57, 59) est alignée avec une extrémité d'élément greffon (12, 13).

5. Prothèse endovasculaire (50) selon la revendication 1, dans laquelle ledit élément formant greffon (11) est fixé audit premier élément de support (51) et audit deuxième élément de support (52) au moyen d'éléments de fixation (30, 31) choisis dans le groupe comprenant les crochets, les éléments perçants, les pinces, les sutures, les agrafes, les adhésifs et les éléments pouvant être recourbés.

6. Prothèse endovasculaire (50) selon la revendication 1, dans laquelle au moins une section dudit premier élément de support (51) et au moins une section dudit deuxième élément de support (52) sont fixées à une surface intérieure dudit élément formant greffon (11), la surface intérieure dudit élément formant greffon (11) définissant une lumière d'élément greffon (14), ou au moins une section dudit premier élément de support (51) et au moins une section dudit deuxième élément de support (52) sont fixées à une surface extérieure dudit élément formant greffon tubulaire (11).

7. Prothèse endovasculaire (50) selon la revendication 1, dépourvue d'élément intermédiaire connecté entre ledit premier élément de support (51) et ledit deuxième élément de support (52).

8. Prothèse endovasculaire (50) selon la revendication 1, comprenant au moins un élément intermédiaire (40) fixé à et s'étendant entre ledit premier élément de support (15, 51) et ledit deuxième élément de support (16, 52) le long d'une surface dudit élément formant greffon (11), et reliant ledit premier élément de support (15, 51) audit deuxième élément de support (16, 52).

9. Prothèse endovasculaire (50) selon la revendication 1, dans laquelle ledit élément formant greffon (11) comprend un matériau choisi dans l'ensemble constitué de tissu, tissu autologue, tissu hétérologue, péricarde, péricarde bovin, péricarde porcin, valve aortique, veines, artères, matériaux polymères, polytétrafluoroéthylène, polytétrafluoroéthylène expansé et polyesters.

10. Prothèse endovasculaire (50) selon la revendication 1, dans laquelle lesdits éléments de support (51, 52) ont une longueur qui ne dépasse pas 50 % de la longueur de l'élément formant greffon (11).

11. Prothèse endovasculaire (50) selon la revendication 1, dans laquelle lesdits éléments de support (51, 52) ont une longueur d'environ 0,5 mm à environ 300 mm.

12. Prothèse endovasculaire (50) selon la revendication 1, dans laquelle chacun desdits éléments de support (51, 52) comprend un corps substantiellement tubulaire ayant une configuration comprimée radialement et une configuration expansée radialement.

13. Prothèse endovasculaire (50) selon la revendication 1, comprenant au moins un élément formant oeillet (27) sur un dit élément de support (51, 52), ledit au moins un élément formant oeillet (27) comportant une ouverture (29) conçue pour recevoir un élément de fixation (30) pour fixer ledit élément formant greffon (11) sur ledit élément de support (51, 52).
